# EUROPÄISCHE PATENTSCHRIFT

(11) **EP 1 503 729 B1**
(45) Veröffentlichungstag und Bekanntmachung des Hinweises auf die Patenterteilung: **17.03.2010**
(21) Anmeldenummer: 03720549.9
(22) Anmeldetag: 03.05.2003
(51) Int. Cl.: A61K 8/06, A61K 8/23, A61K 8/27, A61Q 19/00, A61P 17/00, A61P 17/02, A61P 17/06, A61P 17/10, A61K 33/04, A61K 33/30, A61K 31/095

(54) **ZUSAMMENSETZUNG UND DEREN VERWENDUNG ALS PHARMAZEUTISCHE ODER KOSMETISCHE FORMULIERUNG ZUR ÄUSSERLICHEN ANWENDUNG**
COMPOSITION AND THE USE THEREOF AS A PHARMACEUTICAL OR COSMETIC FORMULATION FOR EXTERNAL USE
COMPOSITION ET SON UTILISATION COMME FORMULATION PHARMACEUTIQUE OU COSMETIQUE POUR APPLICATION EXTERNE

(30) Priorität: 10.05.2002 DE 10220868
(43) Veröffentlichungstag der Anmeldung: 09.02.2005
(73) Patentinhaber: Klewinghaus, Maria, 40764 Langenfeld (DE); Klewinghaus, Uwe, 40764 Langenfeld (DE)
(72) Erfinder: Klewinghaus, Maria, 40764 Langenfeld (DE)
(74) Vertreter: Gille Hrabal Struck Neidlein Prop Roos
(86) Internationale Anmeldenummer: PCT/EP2003/004658
(87) Internationale Veröffentlichungsnummer: WO 2003/094881

(56) Entgegenhaltungen:
- EP-A- 0 135 058
- EP-A- 0 281 812
- EP-A- 0 976 381
- EP-A- 1 145 707
- DE-A- 3 704 214
- HU-B- 212 680
- US-A- 5 036 050
- DATABASE CA [Online] CHEMICAL ABSTRACTS SERVICE, COLUMBUS, OHIO, US; WANG, GUOSHEN: "Antibacterial and antiinflammatory compositions for skin disease" retrieved from STN Database accession no. 131:327517 XP002249213 & CN 1 150 022 A (PEOP. REP. CHINA) 21. Mai 1997 (1997-05-21)
- "Rote Liste 2000, ISBN 3-87193-218-3, 32282, 85 033/34, 85 038" StartDateMarker 2000, EndDateMarker ISBN 3-87193-218-3 32282, 85 033/34, 85 038

## Beschreibung

Die Erfindung betrifft eine Zusammensetzung zur Behandlung von Ekzemen.

Im Stand der Technik sind eine Vielzahl schwefelhaltiger topischer Formulierungen bekannt (s. z.B. Cetzsch-Lindenwald "Salben und Salbengrundlagen", S.161 ff, Springer Verlag 1939).

Die DE-A-3704214 offenbart eine Aknesalbe, die 89,2 bis 97,2 Gew.-% pasta zinci oleosa (22 bis 28 %-ig) enthält. Diese Salbe enthält demzufolge jedoch mindestens 64,2 Gew.-% eines Öls. Tatsächlich liegt der Öl-Gehalt sogar noch wesentlich höher, da die maximale Menge der übrigen Komponenten 3,8 Gew.-% beträgt, so dass die Aknesalbe mindestens 96,2 Gew.-% der pasta zinci oleosa enthalten muß, entsprechend einem Öl-Gehalt von 69,3 Gew.-%. Die Salbe enthält kein Wasser. Eine solche öl-basierende Salbe führt jedoch zu einem starken Austrocknungseffekt der Haut. Dadurch wird die Haut anfälliger für Hautprobleme bzw. Hauterkrankungen, so dass die Aknesalbe keine nachhaltige Verbesserung des Hautzustandes bewirkt, und unter Umständen sogar des Zusatzes eines Antibiotikums bedarf. Weiterhin führt der Auftrag dieser öl-basierenden Salbe aufgrund des Zinkoxid-Gehaltes sowie des schlechten Einziehens In die Haut zu einer auffälligen Weißfärbung der Haut, weshalb die Aufgabe der DE-A-3704214 insbesondere auch darin bestand, eine mit Kosmetikfarbstoffen abmischbare Formulierung herzustellen.

Weiterhin betrifft die EP-B1-0135058 ein Verfahren zur Herstellung einer Trägermasse für kosmetische und/oder pharmazeutische Cremes, mit Extrakten aus spurenelementreichen Kräutern. Die darin beschriebene Trägermasse weist einen relativ hohen Gehalt an kolloidalem Schwefel von 5 bis 15 Gew.-% auf, der wesentlich für die Wirkung der Trägermasse ist, die Aufnahme der Wirkstoffe durch die Haut zu ermöglichen. Der Trägermasse wird ein Sud oder Extrakt aus bestimmten, nicht näher bezeichneten spurenelementreichen Kräutern zugemischt, um eine Creme zu erhalten, die zur Behandlung von Stoffwechselstörungen der Haut, wie z. B. Schuppenflechte, Schweißfuß, Akne und Porenverstopfung verwendet werden kann.

Weiterhin ist aus Cetzsch-Lindenwald "Salben und Salbengrundlagen", S.163, Springer Verlag 1939, eine schwefelhaltige Salbe, die sogenannte "Cathaminsalbe" bekannt, die 5 % kolloidalen Schwefel und 10 % Zinkoxid in neutraler Salbengrundlage enthält. Die Salbe wurde für die Behandlung von Ekzemen eingesetzt. Sie wird heute nicht mehr hergestellt, wobei der Grund hierfür nicht mitgeteilt wurde.

Die Erfinder der vorliegenden Patentanmeldung stellten nun fest, dass die Anwendung der vorstehend beschriebenen Formulierungen aus dem Stand der Technik unter Umständen zu Problemen, wie dem Auftreten von starken Hautirritationen führen kann, was dazu führt, dass die Therapie abgebrochen werden muß. Sie nahmen daher Untersuchungen vor, mit dem Ziel diese Probleme der Formulierungen aus dem Stand der Technik zu lösen. Dabei zeigte sich überraschend, dass wenn man Schwefel In einem bestimmten, geringen Mengenanteil verwendet, die erfindungsgemäße Zusammensetzung entweder eine sofortige Linderung des Hautleidens bewirkt oder insbesondere bei der Behandlung akuter Zustände nach einem vorübergehend auftretenden entzündlichen Prozeß von kurzer Dauer, bei dem sich die Haut von entzündlichen Stoffen und Schlackenstoffen befreit, eine Beschwerdefreiheit der betroffenen Hautpartien bewirkt.

Es war weiterhin völlig überraschend, dass mit der erfindungsgemäßen Zusammensetzung ausgezeichnete Wirkungen bei der Behandlung von allergisch bedingten Hautreizungen, atopischer Dermatitis, atopischem Ekzem oder endogenem Ekzem erzielt werden, gleichzeitig jedoch unerwünschte Wirkungen ausbleiben. Probleme wie das Austrocknen der Haut treten mit der erfindungsgemäßen Zusammensetzung In deutlich geringerem Ausmaß auf. Auch lässt sich die erfindungsgemäße Zusammensetzung problemlos auf die Haut auftragen, wobei die Creme gut in die Haut einzieht, so dass sie sich ohne weiteres auch für den großflächigeren Einsatz eignet.

Die vorliegende Erfindung stellt somit eine neue Zusammensetzung,

Die Erfindung betrifft somit eine Zusammensetzung gemäß Anspruch 1.

Die Fettsubstanzen können aus üblichen Fetten, Ölen und Wachsen und Mischungen davon ausgewählt werden, wie gehärtetes Erdnussöl, halbsynthetische Fette, Fettsäuren, Fettsäureestern, Fettalkoholen, Vaseline, Paraffin, Lanolin, hydriertem Lanolin, acetyliertem Lanolin, Bienenwachs, Waltrat, Wollwachsöl usw. Die Öle können insbesondere unter den tierischen, pflanzlichen, mineralischen oder synthetischen Ölen und insbesondere hydriertem Palmöl, hydriertem Ricinusöl, Vaselineöl, Paraffinöl, Purcellinöl, gegebenenfalls flüchtigen Siliconölen und Isoparaffinen ausgewählt werden.

Die Fettsubstanzen enthalten pflanzliche Öle, wie zum Beispiel Sonnenblumenöl mit seinem hohen Gehalt an essentiellen Fettsäuren.

In einer besonderen Ausführungsform wird die Fettsubstanz aus Fettsubstanzen ausgewählt, die mindestens eine mehrfach (d.h. mindestens zweifach) ungesättigte Fettsäure in freier oder chemisch gebundener Form enthalten.

Bevorzugte derartige mehrfach ungesättigte Fettsubstanzen sind pflanzlicher Herkunft, insbesondere pflanzliche Öle, die im wesentlichen aus gemischten Glycerinestern höherer Fettsäuren mit gerader Anzahl von Kohlenstoffatomen bestehen, wie Aprikosenkernöl, Avocadoöl, Baumwollöl, Borretschöl, Distelöl, Erdnussöl, gehärtetes Erdnussöl, Getreidekelmöl, Hanföl, Haselnussöl, Kürbiskernöl, Kokosöl, Leinsamenöl, Lorbeeröl, Mohnöl, Macadamiaöl, Maisöl, Mandelöl, Nachtkerzenöl, Olivenöl, hydriertes Palmöl, Palmöl, Pistazienkernöl, Rapsöl, Rizinusöl, Sanddornöl, Sesamöl, Sojaöl, Sonnenblumenkernöl, Traubenkernöl, Wallnussöl, Weizenkeimöl, Wildrosenöl, Kokosfett, Palmfett, Palmkernfett oder Rüböl.

Die genannten mehrfach ungesättigten Fettsubstanzen können ferner Fettsäuren selbst und deren Derivate, Insbesondere Ester (Glycerinester etc.) einschließen. Die verwendete mehrfach ungesättigte Fettsubstanz enthält mindestens eine mehrfach ungesättigte Fettsäure in freier oder in chemisch gebundener Form. Bevorzugt ist die mehrfach ungesättigte Fettsäure in chemisch gebundener Form, bevorzugter als Triglycerid von Fettsäure(n), ganz besonders bevorzugt In der Form pflanzlicher Fette und/oder Öle enthalten.

Bei den bevorzugt verwendeten Fetten und/oder Ölen, die die mehrfach ungesättigten Fettsäure(n) enthalten, handelt es sich um solche, die einen sogenannten P/S-Quotienten von mindestens etwa 0,5 aufweisen. Werden Mischungen von ungesättigten Fetten und/oder Ölen verwendet, weist mindestens eines der verwendeten Fett und/oder Öl einen P/S-Quotienten von mindestens etwa 1, bevorzugter mindestens etwa 2 auf. Noch bevorzugter weist mindestens eines der verwendeten Fette und/oder Öle einen P/S-Quotienten von mindestens etwa 3, gelegentlich mindestens etwa 5 auf.

Bei dem sogenannten P/S-Quotienten handelt es sich um das Gewichtsverhältnis des Gehalts der mehrfach ungesättigten Fettsäuren zu dem Gehalt der gesättigten Fettsäuren, die In einem Fett und/oder Öl enthalten sind. Der P/S-Quotient für ein gegebenes Fett und/oder Öl wird bestimmt durch die Bestimmung des prozentualen Gehalts der mehrfach ungesättigten Fettsäuren, bezogen auf die Gesamtmenge der Fettsäuren und die Bestimmung des prozentualen Gehalts der gesättigten Fettsäuren, bezogen auf die Gesamtmenge der Fettsäuren, und Bildung des Quotienten:
"Prozentualer Gehalt der mehrfach ungesättigten Fettsäuren, bezogen auf die Gesamtmenge der Fettsäuren / Prozentualer Gehalt der mehrfach gesättigten Fettsäuren, bezogen auf die Gesamtmenge der Fettsäuren".

Die Bestimmung des Fettsäuregehalts von Fetten und/oder Ölen und die anschließende Berechnung des resultierenden P/S-Quotienten erfolgt dabei wie in P. Schweiger, S. Kerschmann in "Untersuchungen über die Fettsäure- und Tocopherolgehalte von Pflanzenölen". Abschlußbericht über das Arbeitsprojekt 'Pflanzenöle' der Landesanstalt für Pflanzenbau, Forchheim, Kapitel 3.2 beschrieben (s.a. "Rohfettgehalt und Fettsäurezusammensetzung verschiedener Pflanzenöle", Abschlußbericht über das Arbeitsprojekt 'Pflanzenöle' der Landesanstalt für Pflanzenbau, Forchheim). D.h., die Bestimmung der Fettsäuremuster erfolgt mittels Gaschromatographie, wobei die Fettsäuretriglyceride aufgrund ihrer Schwerflüchtigkeit zu Methylestern mit Trimethylsulfonlumhydroxid (TSH) umgeestert werden. Dabei werden 50 mg Öl in einen 5 ml Schliffkolben eingewogen und mit 5 ml n-Heptan versetzt. Dazu werden 0,5 ml TSH-Lösung gegeben und gut durchgeschüttelt und über Nacht in den Kühlschrank gestellt. Überschüssiges Methanol setzt sich dabei am Boden des Kölbchens ab. Von der klaren n-Heptanphase wird 1 ml in den Gaschromatograph eingespritzt. Die Herstellung des Methyllerungsreagenz und der Standardlösung erfolgt wie folgt: Eine 200 mm x 20 mm Glaspürette wird mit einem Glaswollpfropfen versehen und mit 35 ml Ionenaustauscher Amberlite IRA-420, der mit Wasser aufgeschlämmt ist, gefüllt. Danach wird der Ionenaustauscher mit 150 ml 4% NaOH-Lösung sowie 150 ml Wasser gewaschen, bis der Ablauf neutral reagiert. Zuletzt erfolgt noch eine Spülung mit 150 ml Methanol. 4,1 g (0,02 mol) Trimethylsulfoniumiodid werden unter Erwärmen auf 50° C in 60 ml Methanol gelöst und in 5 bis 10 ml Portionen über den Ionenaustauscher gegeben. Zum Schluß wird mit 60 ml Methanol nachgewaschen. Die TSH-Lösung wird im Kühlschrank aufbewahrt.

### GC-Parameter

Die GC-Analysen der Fettsäuren werden unter folgenden Bedingungen durchgeführt:
GC: Sichromat; Detektor: FID; Säule: DB-WAX 60 m x 0.25 mm
Temperaturprogramm: Ofentemperatur 150°C Zeit: 5 min;
Aufheizgeschwindigkeit: 10°C/min
Ofentemperatur: 225°C; Zeit: 12 min; Aufheizgeschwindigkeit: 3°C/min
Ofentemperatur: 250°C; Zeit : 20 min (bei Amaranth und Quinoa 30 min)
Injektortemperatur: 250°C; Detektortemperatur: 300°C
Die GC/MS-Untersuchungen werden durchgeführt mit:
GC: HP 5890 Series II; MS: HP 5972 Mass Selectiv Detektor; Säule: DB 17 30 m x.25 mm

Zur Ermittlung der Retentionszeiten der Fettsäuremethylester werden 3 verschiedene Standardlösungen der Firma ROTH (Rotichrom FO2, Rotichrom FO3 und Rotichrom ME29) verwendet. Die Messungen werden In regelmäßigen Abständen wiederholt. Die ermittelten Retentionszeiten nehmen innerhalb von 6 Monaten erfahrungsgemäß ab. Die Retentionszeit für γ-Linolensäure, Elcosadlensäure, Rizinolsäure, Laurinsäure, Caprylsäure und Caprinsäure sowie Squalen wird aus Literaturwerten bestimmt und mit GC/MS nachgeprüft.

Die folgende Tabelle zeigt die häufigsten, natürlich vorkommenden Fettsäuren (nach P. Schweiger, S. Kerschmann in "Untersuchungen über die Fettsäure- und Tocopherolgehalte von Pflanzenölen", ibid.):

| Fettsäuren | Chemische Kurzform | Trivialbezeichnung | Chemische Bezeichnung |
|---|---|---|---|
| gesättigt: | C4:0 | Buttersäure | Butansäure |
| | C6:0 | Capronsäure | Hexansäure |
| | C8:0 | Caprylsäure | Oktansäure |
| | C10:0 | Caprinsäure | Decansäure |
| | C12:0 | Laurinsäure | Dodecansäure |
| | C14:0 | Myristinsäure | Tetradecansäure |
| | C16:0 | Palmitinsäure | Hexadecansäure |
| | C18:0 | Stearinsäure | Octadecansäure |
| | C20:0 | Arachinsäure | Eicosansäure |
| | C22:0 | Behensäure | Docosansäure |
| | C24:0 | Lignocerinsäure | Tetracosansäure |
| | C26:0 | Cerotinsäure | Hexacosansäure |
| einfach ungesättigt | C14:1 | Myristoleinsäure | 9:10 Tetradecensäure |
| | C16:1 | Palmitoleinsäure | 9:10 Hexadecensäure |
| | C18:1 | Ölsäure | 9:10 Oktadecensäure |
| | C18:1 | Vaccensäure | 11:12 Oktadecensäure |
| | C18:1 | Petroselinsäure | 6:7 Oktadecensäure |
| | C20:1 | Gadoleinsäure | 9:10 Eicosensäure |
| | C20:1 | | 11:12 Eicosensäure |
| | C22-1 | Erucasäure | 12:14 Docosensäure |
| | C24:1 | Nervonsäure | 15:16 Tetracosensäure |
| zweifach ungesättigt | C18:2 | Linolsäure | 9:1012:13 Octadecadlensäure |
| | C20:2 | | 8:9 11:12 Eicosadiensäure |
| dreifach ungesättigt | C18:3 | α-Linolensäure | 9,12,15 Octadecatriensäure |
| | C18:3 | γ-Linolensäure | 6,9,12 Octadecatriensäure |
| | C20:3 | | 8,11,14 Eicosatriensäure |
| | C20:3 | | 11,14,17 Eicosatriensäure |
| vierfach ungesättigt | C18:4 | Stearidonsäure | 6,9,12,15 Octadecatetraensäure |
| | C20:4 | Arachidonsäure | 5,8,11,14 Eicosatetraensäure |
| | C22:4 | | 7.10,13,16 Docosatetraensäure |
| fünffach ungesättigt | C20:5 | Timnodonsäure | 5,8,11,14,17 Elcosapentaensäure (EPA) |
| | C22:5 | Clupanodonsäure | 4,8,12,15,19 Docosapentaensäure |
| sechsfach ungesättigt | C22:6 | Cervonsäure | 4,7,10,13,16,19 Docosahexaensäure (DHA) |

Bis auf Buttersäure, Capronsäure, EPA und DHA lassen sich alle gelisteten Fettsäuren in Pflanzenölen und -fetten finden. Fettsäuren mit mehr als drei Doppelbindungen kommen jedoch nur in sehr geringen Mengen vor, wobei Stearidonsäure die in Hanföl in einer Konzentration von ca. 1 % vorkommt, eine Ausnahme bildet. In Pflanzenölen häufig vorkommende, mehrfach ungesättigte Fettsäuren sind insbesondere Linolsäure und Linolensäure.

Die P/S-Quotienten können natürlich abhängig von der Herkunft der pflanzlichen Fette und/oder Öle In gewissen Bereichen schwanken. Zu den Ölen mit einem sehr hohem Anteil an mehrfach ungesättigten Fettsäuren (über 65 %), die einen P/S-Quotienten von > 5,5 aufweisen, gehören beispielweise Distelöl, Sonnenblumenöl (normal), Hanföl, Wildrosenöl, Tabaköl, Königskerzenöl, Mohnöl, Leinsamenöl, Nachtkerzenöl und Walnussöl. Die erfindungsgemäße Zusammensetzung enthält bevorzugt mindestens eines dieser Öle.

Zu den Ölen mit einem hohen Anteil an mehrfach ungesättigten Fettsäuren (über 50 bis 65 %), die einen P/S-Quotienten von etwa 3,8 bis 5,5 aufweisen, gehören beispielsweise Traubenkernöl, Schwarzkümmelöl, Borretschöl, Kürbiskernöl, Sojaöl und Weizenkeimöl.

Zu den Ölen mit einem P/S-Quotienten von etwa 2.2 bis 3.8 gehören beispielsweise Rapsöl, Haselnussöl, Erdnußöl, Aprikosenkernöl, Mandelöl, Sonnenblumenöl (high-oleic) und Pistazienöl.

Öle mit einem P/S-Quotienten von < 1 sind beispielweise Olivenöl, Macadamiaöl, Avocadoöl und Sanddornöl.

Die vorstehend genannten mehrfach ungesättigten Fettsubstanzen können in Mischungen mit einfach ungesättigten und gesättigten Fettsubstanzen verwendet werden. Bevorzugt enthält die erfindungsgemäße Zusammensetzung mindestens eine mehrfach ungesättigte Fettsubstanz.

Die Zusammensetzung enthält 5 bis 15 Gew.-% Fettsubstanzen.

Als Zinkoxid wird übliches für kosmetische oder pharmazeutische Formulierungen geeignetes Zinkoxid mit DAB-Qualität eingesetzt.

Die Menge des Zinkoxids in der Zusammensetzung beträgt 20 bis 30 Gew.-%, bevorzugter 26 bis 29 Gew.-%.

Ein Zinkoxidgehalt von 20 bis 30 Gew.-% ist besonders vorteilhaft, weil damit in der erfindungsgemäßen Zusammensetzung bzw. Formulierung eine besonders hautberuhigende Wirkung erzielt wird.

Die erfindungsgemäße Zusammensetzung enthält weiterhin elementaren Schwefel, in wobei die Gesamtmenge des in der Zusammensetzung Form von elementarem Schwefel und/oder Schwefelverbindungen enthaltene Schwefels weniger als 4,5 Gew.-%, bezogen auf den enthaltenen Schwefel beträgt.

Der Ausdruck "enthält weniger als" meint, dass die Zusammensetzung zwingend Schwefel enthält, der Schwefelgehalt also größer als 0 Gew.-% sein muß.

Ein Schwefelgehalt von mehr als 4,5 Gew.-% kann wie oben erwähnt zum Auftreten von starken Hautirritationen, mithin zu einem Abbruch der Therapie führen.

Die erfindungsgemäße Zusammensetzung enthält bevorzugt weniger als etwa 3 Gew.-%, bevorzugter weniger als etwa 2 Gew.-% Schwefel in der Form von elementarem Schwefel. In bestimmten Fällen beispielsweise In der homöopathischen Behandlung kann der Schwefelgehalt auch bevorzugt unterhalb von 1 Gew.-% liegen.

Als Schwefelquelle dient in der erfindungsgemäßen Zusammensetzung elementarer Schwefel. Dabei kann es sich vorzugsweise um üblichen, für kosmetische oder pharmazeutische Formulierungen geeigneten elementaren Schwefel in verschiedenen Formen handeln, wie beispielsweise Netzschwefel, Schwefel vulkanischen Ursprungs, gemahlener S8-Schwefel, in-situ aus stöchiometrischen Mischungen aus Sulfiden und Sulfiten, vornehmlich in Form der Natrium- und/oder Kallumverbindungen, hergestellter Schwefel und kolloidaler Schwefel. Bevorzugt ist die Anwendung von kolloidalem Schwefel.

Insbesondere bei der homöopathischen Anwendung können auch Lösungen von elementarem Schwefel zum Einsatz kommen.

Die erfindungsgemäße Zusammensetzung enthält weiterhin Wasser. D.h., es handelt sich bei der erfindungsgemäßen Zusammensetzung um eine Emulsionszusammensetzung. Bei dem eingesetzten Wasser handelt es sich um übliches, für die Herstellung kosmetischer oder pharmazeutischer Formulierungen verwendetes Wasser.

Der Wassergehalt der erfindungsgemäße Zusammensetzung liegt bevorzugt bei etwa 40 bis 65 Gew.-%, bevorzugter 50 bis 60 Gew.-%.

Die erfindungsgemäße Zusammensetzung enthält gegebenenfalls mindestens einen Emulgator. Dabei kann es sich um einen für kosmetische und pharmazeutische Emusionsformullerungen üblichen Emulgator handeln. Bevorzugt werden Emulgatoren auf pflanzlicher Basis verwendet. Ein Beispiel stellt Cetylstearylalkohol dar.

Die erfindungsgemäße Zusammensetzung sowie die daraus hergestellten Formulierungen liegen als Wasser-in-Öl-(W/O)-Emulsion oder als Öl-In-Wasser-(O/W)-Emulsion vor. Bevorzugt ist eine Öl-in-Wasser-(O/W)-Emulsion.

Die erfindungsgemäße Zusammensetzung, die lediglich die oben beschriebenen Bestandteile, also mindestens eine Fettsubstanz, Zinkoxid, elementarer Schwefel, Wasser und gegebenenfalls mindestens einen Emulgator enthält, kann als solche bereits als pharmazeutische oder kosmetische Formulierung zur äußerlichen Anwendung verwendet werden, oder sie kann weitere pharmazeutische oder kosmetische wirksame Bestandteile enthalten. Als weitere pharmazeutische bzw. kosmetische Wirkstoffe neben Zinkoxid und elementarem Schwefel kommen zum Beispiel Vitamine, wie Vitamin E, Allantoin, und pflanzliche Extrakte in Betracht.

Die erfindungsgemäße Zusammensetzung wird zweckmäßig durch ein Verfahren hergestellt, bei dem die oben genannten Bestandteile zusammengegeben und bei Temperaturen von etwa 60 bis 200°C emulgiert werden.

Die Erfindung betrifft weiterhin die Verwendung der erfindungsgemäßen Zusammensetzung als pharmazeutische oder kosmetische Formulierung zur äußerlichen Anwendung, insbesondere zur Behandlung von allergisch bedingten Hautreizungen, atopischer Dermatitis, ekzematlschen Erkrankungen aller Art, wie atopisches Ekzem oder endogenes Ekzem. Besonders geeignet ist die erfindungsgemäße Zusammensetzung zur Behandlung von Ekzemen.

Eine pharmazeutische bzw. kosmetische Wirkung wird dabei in den genannten indikationsgebieten bereits mit der lediglich aus Fettsubstanz, Zinkoxid, elementarem Schwefel, Wasser und gegebenenfalls Emulgator bestehenden Zusammensetzung erreicht. Der Zusatz weiterer pharmazeutisch oder kosmetisch wirksamer Bestandteile, wie beispielsweise Vitamine, Allantoin und/oder pflanzlichen Extrakte, kann jedoch zu einer weiteren Verbesserung der Wirkung in den genannten Indikationen führen. Die weiteren pharmazeutisch oder kosmetisch wirksamen Bestandteile können in reiner oder in gelöster oder dispergierter Form der erfindungsgemäßen Zusammensetzung in einer Menge von beispielsweise etwa 0,01 bis 2 Gew.-% bezogen auf die erfindungsgemäße Zusammensetzung zugemischt werden.

Daneben kann die lediglich aus Fettsubstanz, Zinkoxid, elementarem Schwefel, Wasser und gegebenenfalls Emulgator bestehende Zusammensetzung auch als Grundlage für pharmazeutische und/oder kosmetische Formulierungen anderer pharmazeutischer und/oder kosmetischer Wirkstoffe in anderen Indikationsgebieten dienen.

Diese Zusammensetzungen liegen als Emulsionen, beispielsweise in der Form einer Creme, einer Lotion, einer dickflüssigen Lotion, eines Gel, einer Milch, einer Paste etc. vor. Dem Fachmann ist bekannt, wie er die verschiedenen Emulsionstypen abhängig vom Herstellungsverfahren und der Zusammensetzung herstellen kann. Besonders bevorzugt liegt die erfindungsgemäße Zusammensetzung in der Form einer Creme oder Paste vor.

Die Zusammensetzung der Erfindung kann neben den eigentlichen pharmazeutischen und/oder kosmetischen Wirkstoffen gegebenenfalls in solchen Formulierungen übliche Zusätze, wie organische Lösungsmittel, wie niedere Alkohole und Polyole, beispielsweise Ethanol, Isopropanol, Propylenglykol, Glycerin und Sorbit, Silicone, Verdickungsmittel, reizlindernde Mittel, Sonnenschutzfilter, Antischaummittel, Hydratisierungsmittel, Parfums, Konservierungsmittel, grenzflächenaktive Stoffe, Füllstoffe, Maskierungsmittel, anionische, kationische, nichtionische oder amphotere Polymere oder deren Gemische, Treibmittel, Mittel zum Ansäuern oder Alkalischmachen, Färbemittel, Pigmente oder Nanopigmente, wie Titanoxid (amorph oder kristallin In Form von Rutil und/oder Anatas), Oxide von Eisen, Zirconium oder Cer, die beispielsweise als Lichtschutzmittel bekannt sind, Aluminiumoxid, und/oder Aluminiumstearat, Lösemittel, wie Alkohole, etc. enthalten.

Bevorzugt enthält die Zusammensetzung jedoch nur die oben genannten wesentlichen Bestandteile, also Fettsubstanz, Zinkoxid, elementarer Schwefel, Wasser, gegebenenfalls Emulgator sowie gegebenenfalls weitere pharmazeutisch oder kosmetisch wirksame Bestandteile.

## Patentansprüche

1. Zusammensetzung
zur Behandlung von Ekzemen,
enthaltend :
- 5 bis 15 Gew.-% mindestens einer Fettsubstanz,
wobei pflanzliches Öl enthalten ist,
- 20 bis 30 Gew. -% Zinkoxid,
- Schwefel in der Form von elementarem Schwefel,
- 40 bis 65 Gew.-% Wasser,
- gegebenenfalls mindestens einen Emulgator,
wobei sich die Mengenangaben auf die Gesamtmenge der Zusammensetzung beziehen,
und wobei die Gesamtmenge des in der Zusammensetzung in Form von elementarem Schwefel und/oder von Schwefelverbindungen enthaltenen Schwefels weniger als 4,5 Gew.-%, bezogen auf den enthaltenen Schwefel, beträgt.

## Claims

1. Composition
for the treatment of eczema,
comprising
- 5 to 15 % by weight of at least one fatty substance, with vegetable oil being included,
- 20 to 30 % by weight of zinc oxide,
- sulfur in the form of elementary sulfur,
- 40 to 65 % by weight of water,
- if required, at least one emulsifier,
wherein the indicated amounts relate to the total amount of the composition,
and wherein the total amount of the sulfur contained in the composition in the form of elementary sulfur and/or of sulfur compounds is less than 4.5 % by weight relative to the sulfur contained.

## Revendications

1. Composition
destinée au traitement des eczémas,
contenant :
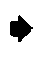 5 à 15 % en poids d'au moins une substance grasse, de l'huile végétale étant contenue,
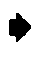 20 à 30 % en poids d'oxyde de zinc,
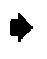 du soufre sous forme de soufre natif,
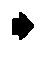 40 à 65 % en poids d'eau,
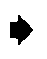 le cas échéant au moins un agent émulsifiant,
les indications de quantité se rapportant à la quantité totale de la composition,
la quantité totale du soufre contenu dans la composition sous forme de soufre natif et/ou de composés de soufre étant inférieure à 4,5 % en poids, par rapport au soufre contenu.
